# EUROPÄISCHE PATENTANMELDUNG

(11) **EP 2 371 270 A1**
(43) Veröffentlichungstag der Anmeldung: **05.10.2011**
(21) Anmeldenummer: 10002727.5
(22) Anmeldetag: 16.03.2010
(51) Int. Cl.: A61B 3/10, A61B 3/103

(54) **Verfahren zur Kompensation und Simulation der (anamorphotischen) Verzeichnung**

(71) Anmelder: Stuetz, Ignaz Alois, 4212 Neumarkt im Muehlkreis (AT)
(72) Erfinder: Stuetz, Ignaz Alois, 4212 Neumarkt im Muehlkreis (AT)
(74) Vertreter: Piermayr, Alexander

(57) **Zusammenfassung**

Bei der subjektiven Brillenglasbestimmung werden Messgläser vor das Auge gebracht, wobei die Probanden die unterschiedlichen refraktiven Wirkungen in ihrer Abbildungsqualität beurteilen sollen. Die Gesetzmäßigkeit der optischen Abbildung bewirkt neben der beabsichtigen Verschicbung der Bildlage auch eine Formveränderung, wobei die anamorphotische Verzeichnung und die Bildfeldwölbung subjektiv stark irritieren. Die Erfindung berechnet diese Formveränderung der in den Strahlengang gebrachten Wirkung und verändert die visuellen Objekte in ihrer Form gegengleich, sodass auf der Netzhaut die Abbildung in der gewohnten Form erscheint. Diese Effekte können die Probanden bei der Beurteilung der Abbildungsvarianten nun nicht mehr stören, die Brillenglasbestimmung wird beschleunigt und präzisiert. Dass diese Optimierung konkrete Brillen nicht aufweisen ist kein Problem, weil diese Verzeichnungen beim Tragen durch Gewöhnung eliminiert werden. Um die optimale Korrektion bei länger bestehenden Refraktionsdefiziten zu ermitteln werden vorhandene neuronale Kompensationen einbezogen. Gegenüber den Messgläsern kann zusätzlich der tatsächliche Seheindruck für unterschiedliche Korrektionsgläser und -arten wie auch für Kontaktlinsen und intraokulare Korrekturen zum Vergleich simuliert werden.

## Beschreibung

### 1. Das technische Gebiet:

Für die Korrektion von Defiziten des Sehvermögens wird in der Optometrie den sogenannten subjektiven Verfahren gegenüber der Ermittlung objektiver Messwerte, die nicht durch Reaktionen oder Bewertungen des Probanden ermittelt werden, der Vorzug gegeben.

### 2. Stand der Technik:

Bei diesen subjektiven, mithin an den Wahrnehmungen des Probanden und den von ihm hierüber getätigten Äußerungen orientierten Verfahren, für die Brillenglasbestimmung werden in systematischer Vorgangsweise die Seheindrücke von ein- und demselben visuellen Objekt bei unterschiedlichen, zunächst sphärischen, später auch zylindrischen und bei Bedarf prismatischen Wirkungen verglichen, also erhoben, ob nun der Seheindruck gegenüber einer ersten Stellung ("ohne") in einer zweiten Stellung ("mit") "besser oder gleich", "besser oder schlechter" bzw. "mit oder ohne" für den Probanden wird.

Verschiedene in jüngerer Zeit patentierte Verfahren interpretieren über Wellenfrontanalyse objektiv ermittelte Werte und simulieren die Auswirkung auf das Sehvermögen (DE102005054691 A1). Oder es werden die refraktiven Wirkungen von Refraktionsdefiziten als Längsfehler, also Bildlagenverschiebung längs der optischen Achse vor bzw. hinter die Netzhaut, mittels modifizierter bildgebender (Laser-)Strahlen zu simulieren versucht (WO 2007/036356 A1).

Durch den Probanden sollen bei der subjektiven Abfrage gezielt nur Brechwertunterschiede beurteilt werden, was allerdings für die Probanden durch sonstige die Wahrnehmung beeinflussende Faktoren schwierig ist: Denn insbesondere durch den oft relativ großen Abstand zum Auge beim Vorhalten von Messgläsern oder Flippen der Kreuzzylindcr entsteht durch die Bildlagenverschiebung längs der optischen Achse nicht nur ein, wie beabsichtigt, im Kontrast verändertes Bild, sondern sozusagen als "Nebenwirkung" über die sogenannte Systemvergrößerung, ein in der Form verändertes Bild. Bei sphärischen Plusgläsern wird das Objekt größer, bei Minusgläsern kleiner. Beim Vorhalten bzw. Flippen des Kreuzzylinders kommt es zu einer äußerst unangenehmen Erscheinung, der "anamorphotischen Verzeichnung": beispielsweise wird aus einem quadratischen visuellen Objekt je nach Achsenlage ein Rechteck oder ein Parallelogramm, ein Kreis wird zu Ellipsen verformt, die je nach Stellung des Kreuzzylinders die unterschiedlichsten Richtungen annehmen.

Verstärkt wird diese Irritation durch die Tatsache, dass sich bei nicht oder nicht vollständig auskorrigierten (insbesondere astigmatischen) Fehlsichtigkeiten in der Netzhaut natürliche Anpassungen als beste Interpretation des bisher nicht optimalen Strahlenganges ausbilden. Diese eingeübten Kompensationen überlagern sich nun mit den plötzlich auftretenden, formverändernden Erscheinungen, sodass dafür empfindsame Probanden die Gewichtung oft auf die gewohnteste oder erwartete Form legen anstatt, wie vom Prüfer gewünscht, auf den besten Kontrast. Für die optimale Korrektion im Alltag ist nämlich die beste Kontrastabbildung entscheidend, und nicht, wie allgemein kolportiert, das beste Auflösungsvermögen für kleine Objekte in Hochkontrast.

Die (anamorphotische) Verzeichnung durch eine vollkorrigierende Brille ist, wenn sie auch hinokular abgeglichen wurde, bei ständigem Tragen im Alltag normalerweise kein Problem, weil die richtige Form ein Erfahrungswert ist. Eine vielleicht anfangs ungewohnte Veränderung durch die neue Brillenglasstärke wird im Laufe von wenigen Tagen bis Wochen sozusagen "geradegebogen". Beim subjektiven Sehtest allerdings kann dieser Effekt stark irritieren. Dies tritt insbesondere bei Anwendung von Kreuzzylindern zutage, sodass stärkere Wirkungen als ± 0,25 dpt ungern bzw. selten eingesetzt werden.

### 3. Beschreibung der Erfindung im Detail:

Notwendig ist die Eliminierung der formverändernden und subjektiv irritierenden Nebenwirkung der Messgläser bei der Brillenglasbestimmung, die insbesondere bei (Kreuz-) Zylindern in Erscheinung treten. Zentraler Punkt der Erfindung ist, unter Einsatz einer nachfolgend genauer beschriebenen technischen Anordnung, die visuellen Objekte berechnet formverändert so "falsch" darzustellen, dass sie, nachdem sie die refraktiv - korrigierende Wirkung der Messgläser durchlaufen haben, in der Form wie vom Probanden erwartet "richtig" auf die Netzhaut abgebildet werden.

Visuelle Objekt können, technisch betrachtet, unterschiedlich präsentiert werden. Sei es durch reelle Objekte auf Tafeln, Bildschirmen oder Projektionen, sei es virtuell durch Projektion ins Auge, durch Beugung und Interferenz oder in beliebiger Kombination dieser Varianten zum Zweck, ein reelles Bild in der Nähe der Netzhaut zu generieren. Um das erfindungsgemäße Ziel zu realisieren, müssen, unabhängig von der Art und Technik der Darstellung der visuellen Objekte, diese derart ausgeführt sein, dass sie sich schnell und synchron mit dem Vorhalten von Gläsern ändern lassen. Dies kann unter optisch-mechanischer Steuerung, unter elektronischer Steuerung optisch-mechanischer Elemente oder durch rein elektronische Steuerung bilderzeugender Verfahren erfolgen. Es geht um eine präzise und zügige Formänderung des oder der betrachteten visuellen Objekte genau gegengleich zu der bekannten (anamorphotischen) Verzeichnung durch die Abbildung.

Die erfindungsgemäße Anordnung besteht in der Praxis darin, dass entweder eine Vorrichtung die Art, Stärke, Position (als Entfernung zum Auge) und Achsenlage bei (Kreuz-)Zylindern der Messgläsern "erkennen" und dann durch eine Koppelung mit einer variablen Darstellung der visuellen Objekte diese ändern kann, oder dass durch entsprechende übergeordnete Anordnung beide Faktoren (die refraktive Wirkungsänderung sowie die Formänderung der visuellen Objekte) gleichzeitig gesteuert werden. Jedenfalls wird je nach vorgehaltenem Glas oder Schaltung, je nach Achsenlage etc. zeitgleich mit diesem Vorgang auch die Form gegenglcich verändert, sodass sich insgesamt für den Probanden ein in der Form "richtiges", unverändertes Bild ergibt.

Damit fällt die irritierende und irreführende Formveränderung weg und der Proband kann nur mehr die unterschiedlichen Bildlagen beurteilen. Werden als visuelle Objekte anstatt der gängigen hochkontrastigen kleinen Optotypen kontrastarmc und damit verbunden auch größere Objekte oder Bilder präsentiert, deren Auflösungskriterium (weit) über der Autlösungsgrenze des Probanden steht, wird nicht mehr die beste Refraktion für das höchste Auflösungsvermögen bei Objekten mit hohem Kontrast, sondern die beste Abbildung für Objekte mit niedrigem Kontrast beurteilt, was normalerweise dem optimalen, blend- und belastungsfreien Sehvermögen im Alltag bei länger dauernden, (von der Beleuchtung her) anspruchsvollen, oder die persönliche Sicherheit betreffenden Sehaufgaben näherkommt.

Gängig werden derzeit verschiedene Korrektionen dadurch verglichen, dass sie zeitlich hintereinander den Probanden offeriert werden. Denkbar ist auch eine Verdoppelung (Vervielfachung) oder gleichzeitige Präsentation mehrerer visueller Objekte in Koppelung mit zugeordneten Strahlengängen unterschiedlicher refraktiver Wirkung, sodass sie von den Probanden simultan beurteilt werden können. Auch bei einem solchen simultanen Verfahren sind die cinzelnen Darstellungen, den jeweiligen Korrektionen und ihren formverändernden Wirkungen entsprechend, in der Form gegengleich darzustellen. In dieser Anordnung ist es dann nicht mehr so eilig, weil statt der sukzessiven eine simultane Entsprechung hergestellt wird, sodass eine wesentlich einfachere, auch rein mechanische Ausführung, ohne automatischelektronische Koppelung der Korrektionsgläser mit den Darstellungsvarianten, realisiert werden kann.

Derzeit ist noch nicht bekannt, wie hoch der Anteil bzw. die Wirkung des neuronal kompensatorischen Bildverarbeitungssystems bei Probanden mit bisher fehlender oder nur teilweiser Korrektion des insbesondere astigmatischen Refraktionsdetizits ist. Es kann sich durch Reihensehtests bei entsprechender Vorgeschichte herausstellen, dass, abgesehen von der relativ einfach berechenbaren vollständigen Formveränderung, ein nach einer zu bestimmmenden komplexeren Funktion korrigierter Wert vorzugeben ist. Dies gilt besonders, wie nachfolgend beschrieben, bei Korrektionen mit Prismengläsern, bei Vergrößerungsdifierenzen durch unterschiedlichen Korrektionsbedarf rechts/links (z.B. durch Anisometropie) im Übergang vom monokularen zum binokularen Sehen sowie beim abschließenden Feinabgleich aller Korrektionsparameter unter binokularen Sehbedingungen:

Die oben beschriebene komplexere Kompensation ist besonders in Betracht zu ziehen, wenn die Erfindung auf die Wirkungen von Prismengläsern erweitert wird, indem erfindungsgemäß ihre anamorphe Wirkung bei der subjektiven Brillenglasbestimmung im vorhinein kompensiert wird. Gerade hier ist die je nach Lichtwellenlänge unterschiedliche Positionsänderung als Farbenquerfehler als folge der bekannten Dispersion zu beachten. Bisher fand in der Praxis kaum Beachtung, dass Prismengläsern bereits monokular eine stark formverändernde Wirkung aufweisen. Bekannter sind die durch binokulare Interpretation subjektiv generierten Effekte von prismatischer Korrektion mit, je nach Basislage, tonnen- oder kissenförmigen Verzeichnungen, veränderter Entfernungseinschätzung, subjektiver Vertiefung bzw. Erhöhung von Objekten auf planen Flächen je nach Farbe sowie "Schwanken" des Raumes bei Kopfdrehung.

Bei der subjektiven Brillenglasbestimmung kommt an einem definierten Punkt der Übergang vom monokularen zum binokularen Sehen. Unter Anwendung gängiger Trennverfahren werden dann nicht nur unterschiedliche visuelle Objekte ins rechte und linke Auge abgebildet, sondern es werden im Sinne der Erfindung diese auch, rechts und links gleich, oder auch unterschiedlich formverändert präsentiert.

Weiters werden unter binokularen Bedingungen individuell eingeübte, ausgleichende Mechanismen ausgelöst. Somit kann die erfindungsgemäße Kompensation mit ihren Parametereinstellungen einen anderen Wert annehmen als 100% bzw. jenem bei monokularer Darstellung. Die erfindungsgemäße Anordnung erlaubt die Anpassung dieser Teilkompensation unter Testbedingungen, sodass auch ermittelt werden kann, bis zu welchem Grad die visuelle Wahrnehmung mit Korrektion als "angenehm" wahrgenommen wird und damit als "erster Einstieg in die Korrektion" zugemutet werden kann (vgl. ganz unten).

Zu beachten ist weiters die mögliche Veränderung der besten Korrektion beim Übergang vom monokularen zum binokularen Sehen. Gängig wird im "binokularen Feinabgleich" die Sphäre zur Herstellung des Refraktionsgleichgewichtes abgeglichen. Zu beobachten ist auch, wenn dies auch wenig bekannt und von der Stärke her gering ist, dass unter binokularen Bedingungen bzw. nach Entlastung der Fusion durch prismatische Korrektion, die beste Korrektion (Sphäre, Cylinder und Achse) verändert sein kann. Zu empfehlen ist jedenfalls der nochmalige Feinabgleich aller korrigierender Parameter unter binokularen Sehbedingungen bzw. nach Änderung einer Prismenkorrektion, wofür sich die Erfindung hervorragend eignen sollte.

Bekannt ist auch, dass bei unterschiedlichem Korrektionsbedarf rechts/links (Anisometropie) Vergrößerungsdifferenzen entstehen. Es sind Messverfahren bekannt, um die Größe dieser sogenannten Aniseikonie zu messen. Solche Messungen werden aber in der Praxis außer im wissenschaftlichen Bereich kaum durchgeführt. Die Erfindung erlaubt in dieser Anwendung nun, durch (teilweise) formkompensierte Darstellung der Sehobjekte rechts/links die Auswirkung auf das Subjekt abzuschätzen. Erweitert um den anamorphotischen Vergrößerungs(differenz)anteil kann nun in der Praxis der notwendige externe Kompensationsanteil durch entsprechende Wahl der (teilweisen) Korrektion, spezieller Brillengläser ermittelt sowie Vor- bzw. Nachteile anderer Korrektionsarten, wie etwa Kontaktlinsen vorgeführt werden.

Messgläser sind gängig sehr dünn und aus Materialen bester Abbildungsqualität hergestellt. Die ermittelte Korrektion muss letztendlich in ein konkretes Brillenglas bzw. ein Brillenglaspaar mit kombinierter Wirkung und Positionierung (Mittendicke, Durchmesser, Krümmung, Zentrierung, Verkippung, Hornhautscheitelabstand, Durchbiegung etc.) übernommen werden, eventuell finden dünne, hochbrechende Glasmaterialien mit höherer Dispersion Verwendung. In einer weiteren Anwendung ist es mit der Erfindung möglich, im voraus die berechenbare Abbildungswirkung des endgültigen Korrektionsglases, zu simulieren wobei z.B. die Dispersion zu je nach Lichtwellenlänge unterschiedlichen Formveränderung (sogenannter Farbenquerfehler) führt, Damit können die Seheindrücke verschiedener Brillengläser insbesondere im Unterschied zu den Messgläsern nach den variablen Parametern wie Hornhautscheitelabstand, Brillenglasdicke, Glasgeometrie, Material und Oberflächenvergütung verglichen werden, indem erfindungsgemäß die berechenbare veränderte Abbildung durch die endgültige Korrektion nun im vorhinein hergestellt wird: durch die formveränderte Darstellung der Sehobjekte in Kombination mit den jetzt im Strahlengang befindlichen Messgläsem entsteht auf der Netzhaut jene Abbildung, wie sie nachher mit der Brille auf der Netzhaut sein wird.

Die Simulation von konkreten Korrektionen ist nicht auf Brillen beschränkt, sondern auch auf Kontaktlinsen und operativ-refraktive Verfahren anwendbar. Gängig geschieht die Umrechnung nur in Bezug auf die refraktive Wirkung. Mit der Erfindung ist es möglich, zur Simulation der Abbildung von Kontaktlinsen oder jener von operativen Korrekturen (soweit prognostizierbar) die Sehobjekte (auch je nach Lichtwellenlänge unterschiedlich) formverändert zu präsenticren, Ein Vergleich verschiedener Ausführungen in ihrer Abbildungsqualität, wobei möglichst alltägliche Sehsituationen hergestellt werden sollten, erleichtert die Entscheidung der Probanden. Die erfindungsgemäß formverändert-kompensierte Darstellung von visuellen Objekten samt simulierter Abbildungsqualität zum Vergleich konkreter Korrektionen ist völlig unabhängig davon, auf welche Art reelle Bilder in die Nähe der Netzhaut gebracht werden. Allgemein gesprochen werden ja nur Photonen in ungleichmäßiger Energieverteilung von den Netzhautrezeptoren verarbeitet - das "Bild" entsteht im Sehzentrum des Gehirns! Außer der Anordnung mit reellen Objekten im Raum, über Bildschirme oder Projektoren erzeugt und unter Verwendung von Messbrillen samt Messgläsern oder einem Phoropter, kann die Bildentstehung nahe der Netzhaut auch über virtuelle Bilder und somit Projektion ins Auge erfolgen. Diese kann teilweise oder vollständig durch diffraktive Bildverarbeitungssysteme ergänzt oder ersetzt werden. Beispielsweise kann durch Holoprafie an beliebigen Orten, also auch auf oder nahe der Netzhaut, ein reelles Bild erzeugt werden. Die Erfindung kann mit allen bilderzeugenden Verfahren, die hier nicht erschöpfend aufgezählt wurden, zur Anwendung gebracht werden.

Es gibt selten aber doch begrilndete Fälle, in denen für einen Probanden nicht die Vollkorrektion des Refraktionsdefizits zweckmäßig ist. Sei es, dass die Korrektion aus praktischen oder gewohnheitsmäßigen Gründen selten getragen wird, sei es, dass eine Vollkorrektion einfach nicht vertragen wird oder wegen eines stufenweisen Aufbaues für einige Zeit eine Teilkorrektion zu empfehlen ist. Mit der erfindungsgemäßen Anordnung kann auch in diesen besonderen Situationen eine für die Klienten optimale, begründete und nachvollziehbare Variante der Vollkorrektion gewählt werden.

## Patentansprüche

1. Verfahren zur Herstellung sowie Präsentation von Sehobjekten zur subjektiven Brillenglasbestimmung sowie zur Simulation der Abbildungswirkung verschiedener Korrektionen, **dadurch gekennzeichnet, dass** visuelle Objekte formverändert, also verzerrt gegenüber der gewohnten, alltäglichen Form, präsentiert werden, wobei diese in Richtung und Größe entweder der formverändemden Wirkung von Korrektionen entspricht, oder in Richtung und Größe zu den refraktiven Wirkungsänderungen durch Messgläser reziprok ist, sodass bei Letzterem die ansonsten gegebene Verzeichung auf der Netzhaut neutralisiert wird.

2. Verfahren nach dem Anspruch 1, **dadurch gekennzeichnet, dass** zur Berechnung dieser formveränderten Darstellung die Art der Korrektionswirkung, wie etwa die Position der brechenden Flächen vor, auf oder im Auge, deren Form, Mittendicke und Materialeigenschaften, Berücksichtigung findet, dazu auch bei Bedarf die Dispersion in die formveränderte Darstellung der visuellen Sehobjekte Eingang findet, indem diese je nach Lichtwellenlänge unterschiedlich erfolgt (Farbenquerfehler).

3. Verfahren nach den Ansprüchen 1 und 2, **dadurch gekennzeichnet, dass** bei der subjektiven Brillenglasbestimmung, bei der verschiedene Korrektionen (Sphäre, Zylinder, Achse und Prisma) zur Wirkung gebracht werden, diese mit der Formänderung des visuellen Objektes gekoppelt sind, indem erfindungsgemäß die Parameter reziprok zur Verzeichnung der Korrektionswirkung gewählt werden.

4. Verfahren nach den Ansprüchen 1 bis 3, **dadurch gekennzeichnet, dass** mit der Änderung der Korrektionswirkung im Abbildungsstrahlengang der Prüfer gleichzeitig aktiv die Formänderung der visuellen Objekte herstellt.

5. Verfahren nach den Ansprüchen 1 bis 3 im Unterschied zu Anspruch 4, **dadurch gekennzeichnet, dass** die Gleichzeitigkeit der refraktiven Wirkungsänderung des Abbildungsstrahlenganges einerseits und der Formänderung der visuellen Objekte andererscits automatisch durch entsprechend gekoppelte technische Ausgestaltung des Präsentation- und Prüfsystems erfolgt, wobei diese Koppelung durch mechanische, optische, elektronische oder kombinierte Steuerungssysteme erfolgen kann.

6. Verfahren nach den Ansprüchen 1 bis 3 im Unterschied zu Anspruch 4 und 5, **dadurch gekennzeichnet, dass** bei Brillenglasbestimmungen, bei denen gleichzeitig visuelle Objekte mit unterschiedlichen Refraktionswirkungen auf die Netzhaut abgebildet werden, die einzelnen visuellen Objekte je nach ihrem zugehörigen Abbildungsstrahlengang in der Form individuell verändert dargestellt werden.

7. Verfahren nach den Ansprüchen 1 bis 6, **dadurch gekennzeichnet, dass** die erfindungsgemäße formveränderte Darstellung von Sehobjekten durch Berechnung der Verzeichnung der Prüflinsen und Korrektionswirkungen nicht exakt reziprok vorgenommen wird sondern, unter Berücksichtigung neuronal-kompensatorischer Vorgänge, mit entsprechend angepassten Faktoren und Parametern durchgeführt wird.

8. Verfahren nach dem Ansprüchen 1 bis 7, **dadurch gekennzeichnet, dass** die erfindungsgemäße formveränderte Darstellung von Sehobjekten bei binokularen Prüf- und Optimierungsverfahren zum Einsatz kommt, sodass unter Einsatz gängiger Trennverfahren bzw. unterschiedlicher Projektionen ins rechte und linke Auge auch jeweils unterschiedliche Formveränderungen der Sehobjekte gewählt werden können.

9. Verfahren nach dem Ansprüchen 1 bis 8, **dadurch gekennzeichnet, dass** die erfindungsgemäße formveränderte Darstellung von Sehobjekten bei binokularen Prüf- und Optimierungsverfahren zum Einsatz kommt, und für formkompensierte Darstellung der Objekte zur Parameteranpassung zusätzlich binokular-kompensatorische Vorgänge der neuronalen Bildverarbeitung, wie Kompensationen von Vergrößcrungsdifferenzen, bei Bedarf unter Berücksichtigung anamorphotischer Seitendifferenzen, sowie die Form- und Raumwahrnehmung verändernde Wirkung von Prismenkorrektionen einbezogen werden.

10. Verfahren nach den Ansprüchen 1 und 2, **dadurch gekennzeichnet, dass** die Abbildung von konkreten Brillengläsern simuliert wird, insbesondere in ihrer formverändernden Wirkung durch ihre Form, Abstand vom Auge, Positionierung und Materialwahl, mithin ihrer gegenüber den Messgläsern veränderten Abbildungswirkung insbesondere bzgl. Verzeichnung und chromatischer Aberration (Farbenquerfehler), unter Einbeziehung des Pupillendurchmessers und gegebenenfalls vorhandener oder zu erwartender, neuronaler Kompensationen.

11. Verfahren nach den Ansprüchen 1 und 2, **dadurch gekennzeichnet, dass** die Abbildungswirkung von Kontaktlinsen simuliert wird, insbesondere in ihrer formverändernden Wirkung durch ihre Form, Position und Bewegung, Materialwahl, Position und Anpassung am Auge, mithin ihrer gegenüber den Messgläsern veränderten Abbildungswirkung insbesondere bzgl. Verzeichnung und chromatischer Aberration (Farbenquerfehler) unter Einbeziehung des Pupillendurchmessers und gegebenenfalls vorhandener oder zu erwartender neuronaler Kompensationen.

12. Verfahren nach den Ansprüchen 1 und 2, **dadurch gekennzeichnet, dass** die Abbildungswirkung von corneal-refraktiven Eingriffen oder intraokularen Korrekturen, soweit präoperativ berechenbar, insbesondere in ihrer formverändernden Wirkung durch ihre Form, Positionierung im Auge und Materialwahl bzw. -abtrag, mithin ihrer gegenüber den Messgläsern veränderten Abbildungswirkung insbesondere bzgl. Verzeichnung und chromatischer Aberration (Farbenquerfehler) unter Einbeziehung des Pupillendurchmessers und gegebenenfalls vorhandener oder zu erwartender neuronaler Kompensationen simuliert wird.

13. Verfahren nach dem Ansprüchen 1 bis 12, **dadurch gekennzeichnet, dass** die Darstellung über reelle visuelle Objekte, insbesondere be- und durchleuchtete Tafeln, Bildschirme und Projektoren, erfolgt.

14. Verfahren nach dem Anspruch 1 bis 12, **dadurch gekennzeichnet, dass** die Entstehung des reellen Netzhautbildes in der Nähe der Netzhaut über virtuelle visuelle Objekte und somit Projektion ins Auge oder mittels Diffraktion bzw. Interferenz oder aber durch Kombination dieser Generierungstechniken erfolgt, wobei die erfindungsgemäße Formvcränderung durch das virtuelle Objekt, durch Manipulation des Abbildungsstrahlcnganges, durch Kombination derselben oder Simulation erfolgen kann.
